# EUROPEAN PATENT SPECIFICATION

(11) **EP 2 369 004 B1**
(45) Date of publication and mention of the grant of the patent: **19.10.2022**
(21) Application number: 09834046.6
(22) Date of filing: 27.09.2009
(51) Int. Cl.: C12P 7/10, C12R 1/72, C12N 1/16, C12N 1/36

(54) **METHOD FOR PRODUCING CELLULOSIC ETHANOL**
VERFAHREN ZUR HERSTELLUNG VON ZELLULOSE-ETHANOL
PROCÉDÉ DE PRODUCTION D'ÉTHANOL CELLULOSIQUE

(30) Priority: 24.12.2008 CN 200810189465
(43) Date of publication of application: 28.09.2011
(73) Proprietor: Angel Yeast Co., Ltd., Hubei 443003 (CN)
(72) Inventor: YU, Xuefeng, Yichang Hubei 443003 (CN); LI, Zhihong, Yichang Hubei 443003 (CN); YU, Minghua, Yichang Hubei 443003 (CN); YAO, Juan, Yichang Hubei 443003 (CN); LI, Zhijun, Yichang Hubei 443003 (CN); LIU, Daiwu, Yichang Hubei 443003 (CN)
(74) Representative: Long, Giorgio
(86) International application number: PCT/CN2009/074249
(87) International publication number: WO 2010/072093

(56) References cited:
- CN-A- 101 041 834
- R. MALESZKA ET AL: "Yeasts that ferment D-cellobiose as well as D-Xylose", BIOTECHNOLOGY LETTERS, vol. 4, no. 2, February 1982 (1982-02), pages 133-136, XP002688065,
- SPINDLER, D.D. ET AL.: 'Thermotolerant Yeast for Simultaneous Saccharification and Fermentation of Cellulose to Ethanol.' APPLIED BIOCHEMISTRY AND BIOTECHNOLOGY. vol. 17, no. 1-3, April 1988, pages 279 - 293, XP008141022
- KARIMI, K. ET AL.: 'Ethanol production from dilute-acid pretreated rice straw by simultaneous saccharification and fermentation with Mucor indicus, Rhizopus oryzae, and Saccharomyces cerevisiae.' ENZYME AND MICROBIAL TECHNOLOGY. vol. 40, no. 1, 06 December 2006, pages 138 - 144, XP025094970
- FREER, S.N. ET AL.: 'Characterization of Cellobiose Fermentations to Ethanol by Yeasts.' BIOTECHNOLOGY AND BIOENGINEERING. vol. 25, no. 2, February 1983, pages 541 - 557, XP008141007
- SPINDLER D D ET AL: "EVALUATION OF THERMOTOLERANT YEASTS IN CONTROLLED SIMULTANEOUS SACCHARIFICATIONS AND FERMENTATIONS OF CELLULOSE TO ETHANOL", BIOTECHNOLOGY AND BIOENGINEERING, WILEY & SONS, HOBOKEN, NJ, US, vol. 34, no. 2, 20 June 1989 (1989-06-20), pages 189-195, XP000071993, ISSN: 0006-3592, DOI: 10.1002/BIT.260340207

## Description

### Technical field

The invention involves the technical field of exploitation of bioenergy, specially involves a method for producing cellulosic ethanol.

### Background art

As petroleum resources exhausting, the oil price rising and the environment deteriorating, to look for renewable resources to replace fossil resource and to exploit clean energy resource have been a focus in the industrial biotechnology field. The exploitation of biomass energy can difuse the dependence on petroleum resources, control carbon dioxide emissions, and promote the development of new industry chains in agriculture, which plays an important role in promoting new country construction in our country.

Fuel ethanol, as a renewable clean resource, has been developing in America and Brazil from 70's last century. The productivity of ethanol has been reached more than 5,000,000 tons per year in our country, wherein the annual yield of fuel ethanol remains about 1,100,000 tons, only second to Brazil, America, ranking the third in the world.

A great deal of maize has been used as a resource to exploit bioenergy, which causes the maize price increase continually, so , after the upsurge of turning foodstuff into ethanol, the exploitation of cellulosic ethanol techniques has become a direction for large-scale commercialization of cellulosic ethanol.

Lignocellulosic material is a largest renewable resource on the earth, including forest, crop straw, the processing remains of agricultural by-products etc., which enters on the important place in the energy flows and material recycle flows of natural ecosystem. It is a potential resource to produce fuel ethanol, thus the possible answer of demand of energy in the future.

Currently, it has prominent issue for the techniques in pilot-scale of cellulosic ethanol. Firstly, the commercialized industrial yeast on the market can ferment glucose and maltose, but not cellobiose. The expensive β- glucosidase, besides cellulase, needs to be added in the production process, in order to avoid the inhibition of cellobiose. Cellulase is needed to hydrolyze cellulose into oligosaccharides. One of these oligosaccharides is cellobiose, which includes 2 glucose molecules and can be further disassembled into glucose. However, cellulase does not have enough β- glucosidase to turn cellobiose disassembled from the hydrolysis of cellulose into glucose. Cellobiose can inhibit endoglucanase and exoglucanase, and reduce the production rate and yield of the whole ethanol in Simultaneous Saccharification and Fermentation (SSF). Furthermore, a great deal of the expensive β- glucosidase need to be supplemented, which results in the remarkably high cost. On the other hand, the yeast to ferment cellobiose has been found, but it has a very low ethanol tolerance.

Secondly, SSF cannot guarantee that the enzymolysis temperature harmonizes with the fermentation temperature. Generally, the enzymolysis temperature is 40-50°C, while the fermentation temperature of Saccharomyces cerevisiae is 28-35°C , thus it has to address the compromise in the process, resulting in excessive amounts of enzyme or yeast, generally with the time in SSF more than 100 hours, lower fermentation efficiency. Thirdly, under the terms of high content of substrate, a large quantity of cellulose is still not converted, the effect of enzymolysis decreases remarkably, and the yield of ethanol is also too low, generally only around 3-5% (v/v).

The current preparation rate and the yield of ethanol fermentation cannot economically meet the requirements of SSF. A national patent ( CN 101130792A) reported a method for preparing fuel ethanol by straw enzymolysis, wherein, fermentation time lasted 10-15 days, the efficiency of enzymolysis was remarkably low. Therefore, there is no feasibility of industrialization. Another national patent ( CN101270372A ) reported a method for concentrating sugar solution in fermentation liquid of cellulosic ethanol, which aimed to resolve the problem of too low sugar concentration after enzymolysis. This shows the current enzymolysis technology cannot meet the requirements of industrialization. Although this patent technology involving concentration can be utilized to change the enzymolyzed sugar concentration, the problem of the low enzymolysis rate of cellulose is not resolved yet, which can cause problems of low utilization rate of raw materials and high production cost. A patent CN 101230359 reported a method for concentrating cellulosic ethanol by a modified polydimethyl siloxane polymer membrane, which also wanted to resolve the problem of too low concentration of fermentation ethanol and high cost of distillation, but there was no way to avoid low efficiency of enzymolysis and fermentation.

The document Spindler et al. (1988), Applied Biochemistry and Biotechnology, vol. 17, no. 1-3, pages 279-293, discloses the results of a screening for thermotolerant yeasts capable of SSF fermentation of cellulose to ethanol. Among the identified yeasts was a strain of Candida lusitaniae which was found to be thermotolerant and to be capable of producing ethanol by SSF in a medium comprising a pure cellulose substrate (Sigmacell 50) at a concentration of 7.5, 10 or 15 % w/v.

### Contents of the invention

The aim of the present invention is to provide a method for producing cellulosic ethanol, by increasing the efficiency of SSF, thus increasing the production rate and the yield of cellulosic ethanol.

To achieve the aim of the present invention, technical solutions are supplied as follows:
A method for producing cellulosic ethanol, which comprises the following steps:
1) adding a medium containing source materials comprising cellulose and/or hemicelluloses to a fermentation reaction vessel:
2) adding cellulase to the fermentation reaction vessel, and inoculating Candida lusitaniae;
3) conducting simultaneous saccharification and fermentation(SSF) with the combined action of cellulase and Candida lusitaniae, and obtaining cellulosic ethanol by separation.

In this method, the origin of the source materials comprising cellulose and/or hemicelluloses in step 1) is renewable lignocellulosic biomass. For example, wheat straw, rice straw and fast growing forest etc. are used as the source materials of cellulosic ethanol. This biomass contains 20-70% cellulose and 10-40% hemicellulose of percent by dry weight of the source materials, and partial lignin. At the same time, it may also include all kinds of biomass which are rich in cellulose and discarded from the environments and living goods.

In the above method, the said medium in step 1) contains 15% - 30% cellulosic and/or hemicellulosic source materials, and it may also contain small amounts of yeast extracts, peptone, antibiotics. The pH value may be controlled to 3.5-6.0. To the medium can be added Tween 80 and ergosterol etc according to the properties of liquids.

In the above method, optionally the cellulase used in step 2) contains endoglucanase, exoglucanase and a small amount of β-glucosidase.

In the above method, the Candida lusitaniae used in step 2) is a yeast that has been adapted to growth in the presence of ethanol and cellobiose. The medium used for adaptation contains cellobiose and proper amounts of ethanol. Three concentration grades, i.e. 5%, 10% and 15%, of cellobiose are used for adaptation, and the yeast is separated by sublines generation after generation, and then ethanol grades, i.e. 1% , 2% and 3%, are used for adaptation, and the dominant strains are isolated.

In the above method, preferably the pH value in SSF is 3.5-6.0, reaction temperature is 35-45°C, preferred 40-45°C. Candida lusitaniae used in SSF in the present invention has a higher ethanol tolerance and a higher ethanol production rate, which can use cellulose and hemicelluloses as source materials and effectively produce cellulosic ethanol with cellulase in SSF. The tests show that Candida lusitaniae has a higher ethanol production rate and a higher yield by the comparison of the fermentation of other yeast using glucose and cellobiose; in addition, compared with other yeasts, Candida lusitaniae has a higher ethanol tolerance.

### Description of figures

Fig.1 is the comparison of productivity of ethanol in SSF between different kinds of yeast and Candida lusitaniae.
Fig.2 is the comparison of ethanol tolerance of different kinds of yeast.

### Mode of Carrying out the Invention

The present invention is detailed by the following modes of carrying out the invention.

The fermentation source materials involved in the present invention contain cellulose and/or hemicelluloses. The origin is renewable lignocellulose biomass, such as wheat straw, rice straw and fast growing forest etc., which are all used as the source materials of cellulosic ethanol. This biomass contains 20-70% cellulose and 10-40% hemicellulose of percent by dry weight of the source materials, and partial lignin. At the same time, it also includes all kinds of biomass which are rich in cellulose and discarded from the environments and living goods.

Cellulase used in the present invention contains endoglucanase, exoglucanase and a small amount of β- glucosidase. Cellulase is used to hydrolyze cellulose into oligosaccharides, such as glucose. One of these oligosaccharides is cellobiose , including 2 glucose molecules, which can be hydrolyzed to glucose. β- glucosidase can turn cellobiose hydrolyzed from cellulose into glucose, but cellulase only contains a little β- glucosidase, thus cellulase itself cannot utilize cellobiose sufficiently.

Candida lusitaniae used in the present invention can utilize cellulose hydrolysis products, cellobiose and glucose, as well as mannose and galactose hydrolyzed from hemicelluloses, and it can also use the above combined sugar and other related sugar to ferment. Herein, disaccharides can be sucrose, maltose, lactose and cellobiose, but not including melibiose and fucose. The monosaccharide hydrolyzed from polysaccharide can also be utilized, for example starch and glucose hydrolyzed from cellulose and other monosaccharides, such as fructose, sorbose, mannose and galactose.

The following illustrates concretely the steps and processes to the effectively produce cellulosic ethanol in SSF involved in the present invention.

### 1. Cultivation of Candida lusitaniae

Candida lusitaniae separated from soil samples enriches in potato agar medium. This yeast strain is purebred in biology, which is identified as Candida lusitaniae (CLAS 5566).

One sample is conserved in the conservation centre of Alan G. Mac Diarmid Research Institute of Renewable Energy, Three Gorges University. The medium used for adaptation contains cellobiose and proper amounts of ethanol. Three concentration grades, i.e. 5%, 10% and 15%, are used for adaptation, the yeast is separated by sublines generation after generation, and then ethanol grades i.e. 1 % , 2% and 3%, are used for adaptation, obtaining advantageous strains by separation.

### 2. Fermentation conditions

### 1 ) Dissolved Oxygen Control of fermentation process

The required scope in the fermentation process is wide. The dissolved oxygen can be either micro-oxygenation in interrupted fermentation, or a little air ventilated through the substrate inoculated in continuous fermentation. Furthermore, it can also be anaerobic fermentation. The required technique will depend on the initial cell density, substrate concentration and inoculation conditions.

### 2 ) The formula of fermentation medium

The medium used for fermentation is a normal medium, which contains proper nitrogen sources, minerals, vitamins and carbon sources. These carbon sources include hexoses (glucose, galactose and mannose) and disaccharides(cellobiose). The medium used for fermentation and inoculation mainly includes 18% cellulose, 1% Angel yeast extract, 2% peptone and 2mg/L antibiotics. pH value is 3.5-6.0. The medium can be added Tween 80 and ergosterol etc. according to the properties of liquids.

### 3 ) The temperature control of fermentation process

The temperature of this fermentation process can range from 28°C to 45°C, however the optimum temperature is 40-45°C.

### 3. Simultaneous saccharification fermentation ( SSF)

In the process of SSF, the saccharification involves the hydrolysis process of cellulose. Cellulase is utilized to hydrolyze cellulose into oligosaccharides. One of these oligosaccharides is cellobiose, which includes 2 glucose molecules, and can be disassembled into glucose.

The yeast strain used in the present invention is Candida lusitaniae, which can ferment cellobiose very well at 42°C, can be used in SSF to provide a faster production rate of wine and a higher ethanol yield , superior to the known other yeasts used for the fermentation of cellobiose.

By comparing the effect of SSF on the final wine production with that of interrupted saccharification fermentations(ISFs), the results in Table 1 show that the utilization rate of cellulose in SSF has been increased by 50% when compared with that of in ISFs, and the yeast efficiency has been increased twice.

**Table 1 The effects of SSF and ISFs on the final wine production**

| Number | SSF process | ISFs process |
|---|---|---|
| saccharification rate | 85.1 % | 58.5% |
| residual reducing sugar (v/v) | 0.15% | 0.235% |
| alcohol degree (v/v, 20°C) | 10.2% | 5.3% |

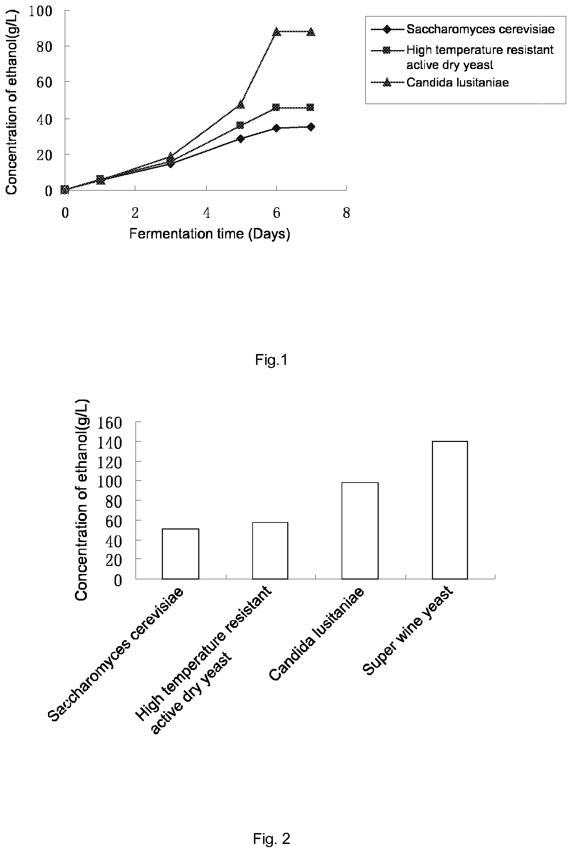

Figure 1 illustrates the data in SSF of different yeast strains. CLAS5566 is compared with other yeast strains, Saccharomyces cerevisiae, and high temperature resistant active dry yeast after 6 days later, the concentration of ethanol utilizing yeast strain CLAS 5566 attained about 90g/L. It is clear to see that it is superior to other yeast strains in rates and yields by comparing with other yeast strains.

Figure 2 shows that at 40°C, CLAS 5566 obtained from 25% cellulose has an ethanol resistant concentration attaining to 100g/L. The ethanol tolerance is remarkably superior to that of Saccharomyces cerevisiae and high temperature resistant active dry yeast, a little lower than that of super wine yeast.

### Scale preparation example 1

Add the medium containing cellulose to a 6L fermentation reaction vessel filled with 2L water, the sample amount reaches 2.5L, to ensure the enough space while inoculating. The medium is mixed in the fermenter containing lipids ( 30ml/L oleic acid ) , and sterilized for 30-40 mins at 120°C. Antibiotics contains 500mg penicillin with the concentration of 10mg/L and 500mg ( 10mg/L ) streptomcycin. pH value is 4.5-5.0. In succession, the cellulase is added and CLAS 5566 special yeast for cellulosic ethanol is inoculated. Cell density is 2×10⁷, the solution is diluted to 3L with water. Enzymes destroy the cellulose into glucose and cellobiose, and the yeast ferments into ethanol. The temperature is controlled at 38°C, simultaneous saccharification ferments for 72 hours, to obtain 89g/L ethanol, and then the ethanol is fractionated from fermentation substrates to obtain cellulosic ethanol.

### Scale preparation example 2

Add the medium containing cellulose to a 20L fermentation reaction vessel filled with 4L water, the sample amount reaches 5.5L, to ensure the enough space while inoculating. The medium is mixed in the fermenter, containing lipids ( ergosterol 5mg/L ) and sterilized for 30-40 min at 120°C. Antibiotics contains 500mg penicillin with the concentration of 10mg/L and 500mg ( 10mg/L ) streptomcycin. pH value is 4.5-5.0. In succession, the cellulase is added, and CLAS 5566 special yeast for cellulosic ethanol is inoculated. Cell density is 2×10⁸, and the solution is dilutee to 5L with water. Enzymes destroy the cellulose into glucose and cellobiose, and the yeast ferments into ethanol. The temperature is controlled at 42°C and simultaneous saccharification ferments for 96 hours, to obtain 98g/L ethanol, and then the ethanol is fractionated from fermentation substrates to obtain cellulosic ethanol.

The method for producing cellulosic ethanol provided by the present invention is introduced in detail above. It needs to point out that the contents described in the modes of carrying out the invention are preferred modes for better implementation of the invention.

## Claims

1. A method for producing cellulosic ethanol, comprising the following steps:
1) adding a medium containing source materials comprising cellulose and/or hemicelluloses to a fermentation reaction vessel;
2) adding cellulase to the fermentation reaction vessel, and inoculating Candida lusitaniae;
3) conducting simultaneous saccharification and fermentation (SSF) with the combined action of cellulase and Candida lusitaniae, and obtaining cellulosic ethanol by separation, wherein said medium in step 1) contains 15%-30% cellulosic and/or hemicellulosic source materials, said source materials containing 20%-70% cellulose and 10%-40% hemicellulose of percent by dry weight of the source materials; wherein, the origin of the source materials of cellulose and/or hemicelluloses in step 1) is renewable lignocellulosic biomass; wherein, Candida lusitaniae used in step 2) is a yeast that has been adapted to growth in the presence of ethanol and cellobiose.

2. The method according to claim 1, wherein, said medium in step 1) contains small amounts of yeast extracts, peptone and antibiotics.

3. The method according to claim 1, wherein the pH value of said medium in step 1) is controlled within the range of 3.5-6.0.

4. The method according to claim 1, wherein, the cellulase in step 2) contains endoglucanase, exoglucanase and a small amount of β-glucosidase.

5. The method according to claim 1, wherein, three concentration grades, i.e. 5%, 10% and 15%, of cellobiose, are used for adaptation, the yeast being separated by sublines generation after generation, and then ethanol grades i.e. 1%, 2% and 3%,are used for adaptation, obtaining advantageous strains by separation.

6. The method according to claim 1, wherein, reaction temperature of the simultaneous saccharification fermentation (SSF) in step 3) is 35-45°C.

7. The method according to claim 6, wherein, the reaction temperature of SSF in step 3) is 40-45°C.

## Patentansprüche

1. Verfahren zur Herstellung von Cellulose-Ethanol, umfassend die folgenden Schritte:
1) Zugeben eines Mediums, das Ausgangsmaterialien enthält, die Cellulose und/oder Hemicellulosen umfassen, in ein Fermentationsreaktionsgefäß;
2) Zugeben von Cellulase zu dem Fermentationsreaktionsgefäß und Inokulieren von Candida lusitaniae;
3) Durchführen einer gleichzeitigen Verzuckerung und Fermentation (SSF) mit der kombinierten Wirkung von Cellulase und Candida lusitaniae und Erhalten von Cellulose-Ethanol durch Abtrennung, wobei das Medium in Schritt 1) 15 % - 30 % Cellulose- und/oder Hemicellulose-Ausgangsmaterialien enthält, wobei die Ausgangsmaterialien 20 % - 70 % Cellulose und 10 % - 40 % Hemicellulose in Prozent des Trockengewichts der Ausgangsmaterialien enthalten;
wobei der Ursprung der Ausgangsmaterialien für Cellulose und/oder Hemicellulosen in Schritt 1) erneuerbare Lignocellulose-Biomasse ist; wobei in Schritt 2) verwendetes Candida lusitaniae eine Hefe ist, die an das Wachstum in der Gegenwart von Ethanol und Cellobiose angepasst ist.

2. Verfahren nach Anspruch 1, wobei das Medium in Schritt 1) geringe Mengen von Hefeextrakten, Pepton und Antibiotika enthält.

3. Verfahren nach Anspruch 1, wobei der pH-Wert des Mediums in Schritt 1) im Bereich von 3,5 - 6,0 geregelt wird.

4. Verfahren nach Anspruch 1, wobei die Cellulase in Schritt 2) Endoglucanase, Exoglucanase und eine geringe Menge von β-Glucosidase enthält.

5. Verfahren nach Anspruch 1, wobei drei Konzentrationsstufen, d. h. 5 %, 10 % und 15 % Cellobiose, für die Anpassung verwendet werden, wobei die Hefe Generation für Generation nach Unterlinien getrennt wird, und dann Ethanolstufen, d. h. 1 %, 2 % und 3 %, für die Anpassung verwendet werden, wobei vorteilhafte Stämme durch Trennung erhalten werden.

6. Verfahren nach Anspruch 1, wobei die Reaktionstemperatur der simultanen Verzuckerungsfermentation (SSF) in Schritt 3) 35 - 45 °C beträgt.

7. Verfahren nach Anspruch 6, wobei die Reaktionstemperatur der SSF in Schritt 3) 40 - 45 °C beträgt.

## Revendications

1. Procédé de production d'éthanol cellulosique, comprenant les étapes suivantes :
1) l'ajout d'un milieu contenant des matières de base comprenant de la cellulose et/ou des hémicelluloses à une cuve de réaction de fermentation ;
2) l'ajout de cellulase à la cuve de réaction de fermentation, et l'inoculation de Candida lusitaniae ;
3) la réalisation d'une saccharification et fermentation simultanée (SSF) avec l'action combinée de cellulase et de Candida lusitaniae, et l'obtention d'éthanol cellulosique par séparation, dans lequel ledit milieu à l'étape 1) contient de 15 % à 30 % de matières de base cellulosiques et/ou hémicellulosiques, lesdites matières de base contenant de 20 % à 70 % de cellulose et de 10 % à 40 % d'hémicellulose en pourcentage de poids sec des matières de base ;
dans lequel l'origine des matières de base de cellulose et/ou d'hémicellulose à l'étape 1) est une biomasse lignocellulosique renouvelable ;
dans lequel la Candida lusitaniae utilisée à l'étape 2) est une levure qui a été adaptée pour une croissance en présence d'éthanol et de cellobiose.

2. Procédé selon la revendication 1, dans lequel ledit milieu à l'étape 1) contient de petites quantités d'extraits de levure, de peptone et d'antibiotiques.

3. Procédé selon la revendication 1, dans lequel la valeur de pH dudit milieu à l'étape 1) est régulée à l'intérieur de la plage de 3.5 à 6.0.

4. Procédé selon la revendication 1, dans lequel la cellulase à l'étape 2) contient de l'endoglucanase, de l'exoglucanase et une petite quantité de β-glucosidase.

5. Procédé selon la revendication 1, dans lequel trois niveaux de concentration, c'est-à-dire 5 %, 10 % et 15 %, de cellobiose sont utilisés pour une adaptation, la levure étant séparée par une génération de sous-lignées après la génération, puis des niveaux d'éthanol, c'est-à-dire 1 %, 2 % et 3 %, sont utilisés pour une adaptation, en obtenant des souches avantageuses par séparation.

6. Procédé selon la revendication 1, dans lequel une température de réaction de la saccharification et fermentation simultanée (SSF) à l'étape 3) est de 35 à 45°C.

7. Procédé selon la revendication 6, dans lequel la température de réaction de SSF à l'étape 3) est de 40 à 45°C.
